# EUROPEAN PATENT SPECIFICATION

(11) **EP 4 495 097 B1**
(45) Date of publication and mention of the grant of the patent: **09.09.2026**
(21) Application number: 24186191.3
(22) Date of filing: 03.07.2024
(51) Int. Cl.: C07C 37/68, C07C 39/19, C07C 37/70, C07C 37/74, C07C 37/82

(54) **FURANOCOUMARIN-FREE BAKUCHIOL COMPOSITIONS AND THEIR METHOD OF PREPARATION THEREOF**
FURANOCOUMARINFREIE BAKUCHIOL-ZUSAMMENSETZUNGEN UND VERFAHREN ZU IHRER HERSTELLUNG
COMPOSITIONS DE BAKUCHIOL SANS FURANOCOUMARINES ET LEUR PROCÉDÉ DE PRÉPARATION

(30) Priority: 03.07.2023 IN 202341044659
(43) Date of publication of application: 22.01.2025
(73) Proprietor: Majeed, Muhammed, Bangalore 560058 (IN); Nagabhushanam, Kalyanam, East Windsor, NJ 08520 (US); Ibrahim Abdul, Muhamad, Bangalore, Karnataka 560058 (IN)
(72) Inventor: Majeed, Muhammed, Bangalore 560058 (IN); Nagabhushanam, Kalyanam, East Windsor, NJ 08520 (US); Ibrahim Abdul, Muhamad, Bangalore, Karnataka 560058 (IN)
(74) Representative: Grünecker Patent- und Rechtsanwälte PartG mbB

(56) References cited:
- WO-A1-2012/105990
- WO-A2-2014/167584
- CN-A- 108 299 453
- CN-A- 112 723 997
- JP-B2- 3 798 107
- US-B2- 11 253 486
- MANOHAR B. ET AL: "Enrichment of bakuchiol in supercritical carbon dioxide extracts of chiba seed (Psoralea corylifolia L.) using molecular distillation-Response surface methodology", vol. 14, no. 1, 1 February 2009 (2009-02-01), KR, pages 112 - 117, XP093231816, ISSN: 1226-8372, Retrieved from the Internet <URL:https://link.springer.com/content/pdf/10.1007/s12257-007-0210-x.pdf> DOI: 10.1007/s12257-007-0210-x

## Description

### FIELD OF INVENTION

The present invention in general relates to compositions comprising bakuchiol, free of furanocoumarins. The invention also covers a method of preparing pure bakuchiol, substantially free of furanocoumarins (psoralen and isopsoralen) from the seeds of *Psoralea sp.*

### BACKGROUND OF THE INVENTION

Bakuchiol, a phenolic compound with one hydroxyl group and an unsaturated hydrocarbon chain on the aromatic ring (STR#1), is one of the main ingredients isolated from *Psoralea sp.* It is generally concentrated in the seeds of *Psoralea corylifolia* L. (Luguminosae) and the aerial parts of *Psoralea glandulosa* L. Bakuchiol has numerous health benefits including antitumorigenic, anti-inflammatory, antioxidative, antimicrobial, and antiviral activities (Nizam et al., Bakuchiol, a natural constituent and its pharmacological benefits, F1000Research 2023, 12:29). It is used in traditional medicines to treat different skin conditions and it is used in many cosmetic formulation for skin lightening and UV protective agent.

Different processes for the isolation and purification of this ingredient have been disclosed in literature (Manohar, B., Udaya Sankar, K. Enrichment of bakuchiol in supercritical carbon dioxide extracts of chiba seed (Psoralea corylifolia L.) using molecular distillation-Response surface methodology. Biotechnol Bioproc E 14, 112-117 (2009); CN114478196A; CN108299453A; CN115417749A; CN115141085A; JP5443754B2; CN113173835A; US20220193002A; CN108653379A). However, most of process fail to isolate pure bakuchiol without the presence of furanocoumarin impurities.

The furanocoumarins - Psoralen (STR#2) and Isopsoralen (STR#3) are also present in *Psoralea sp.* and often are isolated along with bakuchiol. These furanocoumarins present different side effects when administered along with bakuchiol. Both psoralen and isopsoralen cause disturbance in alanine metabolism, glutamate metabolism, urea cycle, glucose-alanine cycle, ammonia recycling, glycine, and serine metabolism pathways and may have more toxic effects on the spleen, adrenal gland, thymus, and liver (Yu et al., Long-Term Exposure of Psoralen and Isopsoralen Induced Hepatotoxicity and Serum Metabolites Profiles Changes in Female Rats, Metabolites. 2019 Nov; 9(11): 263). Psoralen also make the skin more sensitive to sunlight and increase the risk of sunburn, cataracts, and skin cancer. Hence, to reap the full benefits of Bakuchiol, it has be isolated in its pure form, substantially free of the furanocoumarin impurities.

There exist different processes to isolate bakuchiol free the furanocoumarin impurities. JP5443754B2 discloses a process to isolate bakuchiol, substantially free of furanocoumarins by treating a composition containing these impurities with a base (NaOH) thereby converting them to their corresponding carboxylate salts by opening the lactone ring of the furanocoumarins. US11253486B2 also disclosed a composition comprising bakuchiol substantially free of furanocoumarins, however, the content of furnacoumarins were fairly high (Apprx. 100 ppm). There exists an unmet need to isolate bakuchiol using a process that is easy, cheap, scalable and with lesser impurities. The present invention solves the above need by disclosing a process that results in a bakuchiol composition that is substantially free of furanocoumarin impurities (less than 10 ppm). WO-A-2014/167584 discloses the use of silica gel chromatography and solvent removal under vacuum for the purification of bakuchiol. WO-A-2012/105990 and CN 112 723 997 A disclose the use of supercritical fluid extraction and silica gel chromatography for the purification of bakuchiol. JP 3 798107 B2 discloses the use of high vacuum distillation (for deodourisation) and silica gel chromatography for the purification of bakuchiol.

It is the principle object of the invention to disclose a novel process for the isolation of pure bakuchiol (99% w/w) free or substantially free of furanocoumarins (psoralen and isopsoralen).

It is another object of the invention to disclose a composition comprising bakuchiol that is substantially free of furanocoumarins (less than 10 ppm).

The present invention solves the above objects and provides further related advantages.

### SUMMARY OF THE INVENTION

The present inventions discloses a novel process for the isolation of pure bakuchiol (99% w/w) substantially free of furanocoumarins (psoralen and isopsoralen), said process comprising the general steps of :
a) Subjecting the raw material of *Psoralea sp.* to SCFE extraction,
b) Further subjecting the extract from SCFE through High vacuum distillation at controlled temperature / vacuum,
c) Isolating and purifying furanocoumarin free bakuchiol by Silica gel column chromatography. A process according to the invention is defined in claim 1.

The disclosure also discloses a composition comprising bakuchiol that is substantially free of furanocoumarins that is isolated using the process containing the abovementioned steps (such compositions do not form part of the present invention).

### DETAILED DESCRIPTION OF THE MOST PREFERRED EMBODIMENT

### Selected Definitions

All the terms used in this application carry ordinary meaning as known in the prior art unless otherwise specified. Few other specific definitions used in this invention are explained below, which applies throughout this specification. Claims provide broader definition unless and otherwise specified.

Furthermore, the terms "approximately," "approximate," "about," and similar terms generally refer to ranges that include the identified value within a margin of 20%, 10%, or preferably 5%, and any values there between.

As used herein, substantially free of furanocoumarins refer to a composition containing less than 10 ppm of furanocoumarins. And the composition comprising Bakuchiol substantially free of furanocoumarins is branded as Babchiol^{™}.

As used herein, % w/w refers to purity of the component.

As used herein, HVD refers to high vacuum distillation

As used herein, SCFE refers to super critical fluid extraction

In the most preferred embodiment of the invention discloses a process of isolating pure bakuchiol (not less than 99% w/w) as represented in STR#1, substantially free of furanocoumarins from *Psoralea sp.,* comprising steps of:
a) Powdering *Psoralea* sp. plant parts and pulverizing using 1.5 mm mesh and passing through magnetic separator to obtain a coarse powder;
b) Subjecting the enriched extract of step a) to super critical fluid extraction (SCFE) using liquid CO₂ in the presence of an entrainer and a stabilizing agent to obtain two fractions: low volatile compounds-S1 fraction, high volatile compounds-S2 fraction and a spent material (residue);
c) Identifying the compounds in low volatile and high volatile fractions of step b) as bakuchiol, wherein the total yield of bakuchiol in S1 fraction is 5-15% w/w and 0.5 - 3% w/w in S2 fraction;
d) Pooling the S1 and S2 fractions from step b) to obtain bakuchiol enriched extract, substantially free of furanocoumarins;
e) Subjecting the bakuchiol enriched extract of step d) to high vacuum distillation (HVD) at desired temperature with controlled vacuum to obtain different bakuchiol enriched fractions and a pot residue, wherein the bakuchiol content is assayed using HPLC;
f) Pooling the bakuchiol enriched fractions of step e) and passing them through a Silica gel column pre packed with heptane and eluting using a suitable mobile phase and collecting different fractions to get obtain content of bakuchiol (99% w/w) as assayed by HPLC; and
g) Collecting the fractions with bakuchiol content of not less than 99% w/w and further subjecting the fractions to Thin Layer chromatography with suitable solvents as mobile phase with detection under wavelength UV 254 nm, to obtain bakuchiol free or substantially free of furanocoumarins.

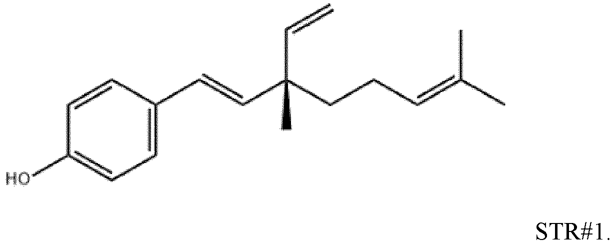

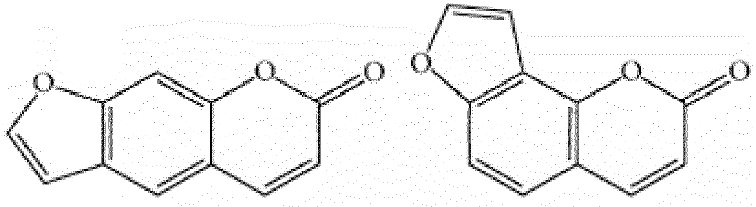

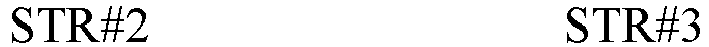

In a related embodiment, the *Psoralea sp.* specifically include *Psoralea corylifolia* L. or *Psoralea glandulosa* L. In another related embodiment the plant parts of *Psoralea sp.* include seeds, stem, leaves, seed pods and fruits. In a preferred embodiment, the plant part is preferably sun-dried seeds. In another preferred embodiment, the furanocoumarins are psoralen and isopsoralen.

In another related embodiment, the conditions of SCFE include, but not limited to, extractor pressure of 250 - 310 bar, separator pressure of 75 - 85 bar with a residence time of 0h -5h. In another related embodiment, the conditions of SCFE preferably include, extractor pressure of 295-305 bar, separator pressure of 78 - 82 bar, 10% w/v ethanol as entrainer and residence time of 3h -5h. In another related embodiment, the stabilizing agent in SCFE is selected from the group consisting of rosmarinic acid, butylated hydroxyanisole, butylated hydroxytoluene, sodium metabisulfite, propyl gallate, cysteine, ascorbic acid and tocopherols. In a related embodiment, the stabilizing agent is preferably rosmarinic acid in the range of 1-90% w/w.

In another related embodiment, the entrainer of step b) is selected from the group consisting of ethanol, methanol, isopropyl alcohol, acetone, chloroform, benzene, hexane, heptane, cyclohexane and combinations thereof. In a preferred embodiment the entrainer of step b) is selected from the group consisting of ethanol, methanol and isopropyl alcohol.

In another embodiment, the temperature in HVD comprised both still temperature and vapour temperature. In a related embodiment, the still temperature as in step h) is between 180-275 °C. In a related embodiment, the still temperature is preferably 195-233 °C, or 233-250 °C or 250-265 °C. In a related embodiment, the vapour temperature as in step h) is between 140-210°C. In a related embodiment, the still temperature is preferably 150-192 °C, or 192-197 °C or 197-205 °C. In a related embodiment, the vacuum pressure as in step h) is 33.3-133.3 Pa (0.25 - 1 mm Hg). In a related embodiment, the vacuum pressure as in step h) is preferably 66.7 Pa (0.5 mm Hg).

In another embodiment, the mobile phase in step f) is selected from the group consisting of, but not limited to, water, methanol, ethanol, propanol, butanol, isopropyl alcohol, hexane, heptane, diethyl ether, chloroform, benzene, ethyl acetate, toluene, dichloromethane, acetone and combinations thereof. In another embodiment, the mobile phase in step f) is selected from the group consisting of n-hexane and ethyl acetate or combinations thereof. In another embodiment, the mobile phase in step i) is selected from the group consisting of heptane and ethyl acetate or combinations thereof. In a preferred embodiment the concentration of ethyl acetate is 0.5-3% (v/v).

In another embodiment, the mobile phase in step g) is selected from the group consisting of, but not limited to, water, methanol, ethanol, propanol, butanol, isopropyl alcohol, hexane, heptane, diethyl ether, chloroform, benzene, ethyl acetate, toluene, dichloromethane, acetone or combinations thereof. In another embodiment, the mobile phase in step j) is selected from the group consisting of n-hexane and ethyl acetate or combinations thereof. In a preferred embodiment the ratio of n-hexane and ethyl acetate is between 2-9 : 1-8. In a preferred embodiment the ratio of n-hexane and ethyl acetate is preferably 7:3. In another embodiment, the mobile phase in step j) is selected from the group consisting of heptane and ethyl acetate or combinations thereof. In a preferred embodiment the ratio of heptane and ethyl acetate is between 2-9 : 1-8. In a preferred embodiment the ratio of heptane and ethyl acetate is preferably 7:3.

In another embodiment, not forming part of the present invention but disclosed herein, there is provided a composition comprising bakuchiol free or substantially free of furanocoumarins, wherein the composition is isolated from *Psoralea sp.,* using a process comprising steps of:
a. Powdering *Psoralea* sp. plant parts and pulverizing using 1.5 mm mesh and passing through magnetic separator to obtain a coarse powder;
b. Subjecting the enriched extract of step a) to super critical fluid extraction (SCFE) using liquid CO₂ in the presence of an entrainer and a stabilizing agent to obtain two fractions: low volatile compounds-S1 fraction, high volatile compounds-S2 fraction and a spent material (residue);
c. Identifying the compounds in low volatile and high volatile fractions of step b) as bakuchiol, wherein the total yield of bakuchiol in S1 fraction is 5-15% w/w and 0.5 - 3% w/w in S2 fraction;
d. Pooling the S1 and S2 fractions from step **c)** to obtain bakuchiol enriched extract, substantially free of furanocoumarins;
e. Subjecting the bakuchiol enriched extract of step d) to high vacuum distillation (HVD) at desired temperature with controlled vacuum to obtain different bakuchiol enriched fractions and a pot residue, wherein the bakuchiol content is assayed using HPLC;
f. Pooling the bakuchiol enriched fractions of step e) and passing them through a Silica gel column prepacked with heptane and eluting using a suitable mobile phase and collecting different fractions to obtain content of bakuchiol (99% w/w) as assayed by HPLC; and
g. Collecting the fractions with bakuchiol content of not less than 99% w/w and further subjecting the fractions to Thin Layer chromatography with suitable solvents as mobile phase with detection under wavelength UV 254 nm, to obtain bakuchiol free or substantially free of furanocoumarins.

In a related embodiment, the *Psoralea sp.* specifically include *Psoralea corylifolia* L. or *Psoralea glandulosa* L. In another related embodiment the plant parts of *Psoralea sp.* include seeds, stem, leaves, seed pods and fruits. In a preferred embodiment, the plant part is preferably sun-dried seeds. In another preferred embodiment, the furanocoumarins are psoralen and isopsoralen.

In another related embodiment, the conditions of SCFE include, but not limited to, extractor pressure of 250 - 310 bar, separator pressure of 75 - 85 bar with a residence time of 0h -5h. In another related embodiment, the conditions of SCFE preferably include, extractor pressure of 295-305 bar, separator pressure of 78 - 82 bar, 10% w/v ethanol as entrainer and residence time of 3h -5h. In another related embodiment, the stabilizing agent in SCFE is selected from the group consisting of rosmarinic acid, butylated hydroxyanisole, butylated hydroxytoluene, sodium metabisulfite, propyl gallate, cysteine, ascorbic acid and tocopherols. In a related embodiment, the stabilizing agent is preferably rosmarinic acid in the range of 1-90% w/w.

In another related embodiment, the entrainer of step b) is selected from the group consisting of ethanol, methanol, isopropyl alcohol, acetone, chloroform, benzene, hexane, heptane, cyclohexane and combinations thereof. In a preferred embodiment the entrainer of step c) is selected from the group consisting of ethanol, methanol and isopropyl alcohol.

In another embodiment, the temperature in HVD comprised both still temperature and vapour temperature. In a related embodiment, the still temperature as in step d) is between 180-275 °C. In a related embodiment, the still temperature is preferably 195-233 °C, or 233-250 °C or 250-265 °C. In a related embodiment, the vapour temperature as in step e) is between 140-210°C. In a related embodiment, the still temperature is preferably 150-192 °C, or 192-197 °C or 197-205 °C. In a related embodiment, the vacuum pressure as in step h) is 33.3-133.3 Pa (0.25 - 1 mm Hg). In a related embodiment, the vacuum pressure as in step h) is preferably 66.7 Pa (0.5 mm Hg).

In another embodiment, the mobile phase in step f) is selected from the group consisting of, but not limited to, water, methanol, ethanol, propanol, butanol, isopropyl alcohol, hexane, heptane, diethyl ether, chloroform, benzene, ethyl acetate, toluene, dichloromethane, acetone and combinations thereof. In another embodiment, the mobile phase in step g) is selected from the group consisting of n-hexane and ethyl acetate or combinations thereof. In another embodiment, the mobile phase in step h) is selected from the group consisting of heptane and ethyl acetate or combinations thereof. In a preferred embodiment the concentration of ethyl acetate is 0.5-3% (v/v).

In another embodiment, the mobile phase in step i) is selected from the group consisting of, but not limited to, water, methanol, ethanol, propanol, butanol, isopropyl alcohol, hexane, heptane, diethyl ether, chloroform, benzene, ethyl acetate, toluene, dichloromethane, acetone or combinations thereof. In another embodiment, the mobile phase in step j) is selected from the group consisting of n-hexane and ethyl acetate or combinations thereof. In a preferred embodiment the ratio of n-hexane and ethyl acetate is between 2-9 : 1-8. In a preferred embodiment the ratio of n-hexane and ethyl acetate is preferably 7:3. In another embodiment, the mobile phase in step k) is selected from the group consisting of heptane and ethyl acetate or combinations thereof. In a preferred embodiment the ratio of heptane and ethyl acetate is between 2-9 : 1-8. In a preferred embodiment the ratio of heptane and ethyl acetate is preferably 7:3.

In another embodiment, not forming part of the present invention, the composition further comprises stabilizing agents, bioavailability enhancers and antioxidants, pharmaceutically or nutraceutically or cosmeceutically accepted excipients and enhancers and suitable for administration in the form of tablets, capsules, syrups, gummies, powders, suspensions, emulsions, chewables, candies, eatables, creams, lotions, ointments, serum, gels or spray.

In another embodiment, not forming part of the present invention, bakuchiol is effectively used in a skin care agent wherein, it reduces fine lines, improves skin texture and skin tone and stimulates skin cell turnover, having vast applications in the cosmetic industry. It is also used as an anti-tumor agent and anti-bacterial agent.

In an embodiment, not forming part of the present invention, the composition can be administered along with other plant ingredients isolated from the different medicinal plants from the group of, but not limited to, *Nigella sativa, Oroxylum indicum, Garcinia cambogia, Garcinia indica, Pterocarpus marsupium, Cyperus rotundus, Coleus forskohlii, Zingiber cassumunar, Withania somnifera, Polygonum cuspidatum, Boswellia serrata, Artocarpus* sp., *Emblica officinalis, Curcuma longa, Terminalia arjuna, Asparagus racemosus* specifically with the ingredients which include, but not limited to, calebin A, oroxylin A, oroxylin B, oroxylin C, withanolides especially with withaferin A and withanone, thymoquinone, thymohydroquinione, scirpusin A, scirpusin B, piceatannol, pterocarposide, forskolin, β-glucogallin, resveratrol, oxyresveratrol, boswellic acids, arjunolic acid, arjunoglucosides, equol and garcinol.

The following examples discloses in detail the preferred embodiments of the invention.

### Example 1: Isolation of Bakuchiol free of furanocoumarins

The present inventions discloses a novel process for the isolation of bakuchiol (99% w/w) free or substantially free of furanocoumarins (psoralen and isopsoralen). It is a three-step process containing the following:
STEP 1: Subjecting the *Psoralea* raw material to SCFE extraction under controlled conditions. The content of bakuchiol in *Psoralea* seed on raw material basis is 4.5-5.5% w/w by HPLC. After subjecting to SCFE the pooled SCFE extract contains 35-40% w/w assay by HPLC
STEP 2: The pooled SCFE extract is subjected to High vacuum distillation under controlled temperature and pressure conditions, wherein the assay of Bakuchiol is enriched from 40% to 66% w/w by HPLC.
STEP 3 : Isolating and purifying bakuchiol free or substantially free of furanocoumarins by Silica gel column chromatography. Bakuchiol 66% w/w is purified/passed through Silica gel column chromatography, to obtain pure bakuchiol 99%w/w by HPLC.

The above steps are disclosed in detail below:
The *Psoralea* raw material is initially extracted with SCFE along with an entrainer to obtain an enriched extract, wherein the entrainer is selected form methanol, ethanol or isopropyl alcohol.

The below flow chart explains the SCFE extraction of bakuchiol form *Psoralea corylifolia* seeds (step 1)
Flow chart No 1: Process for isolating Bakuchiol 99%

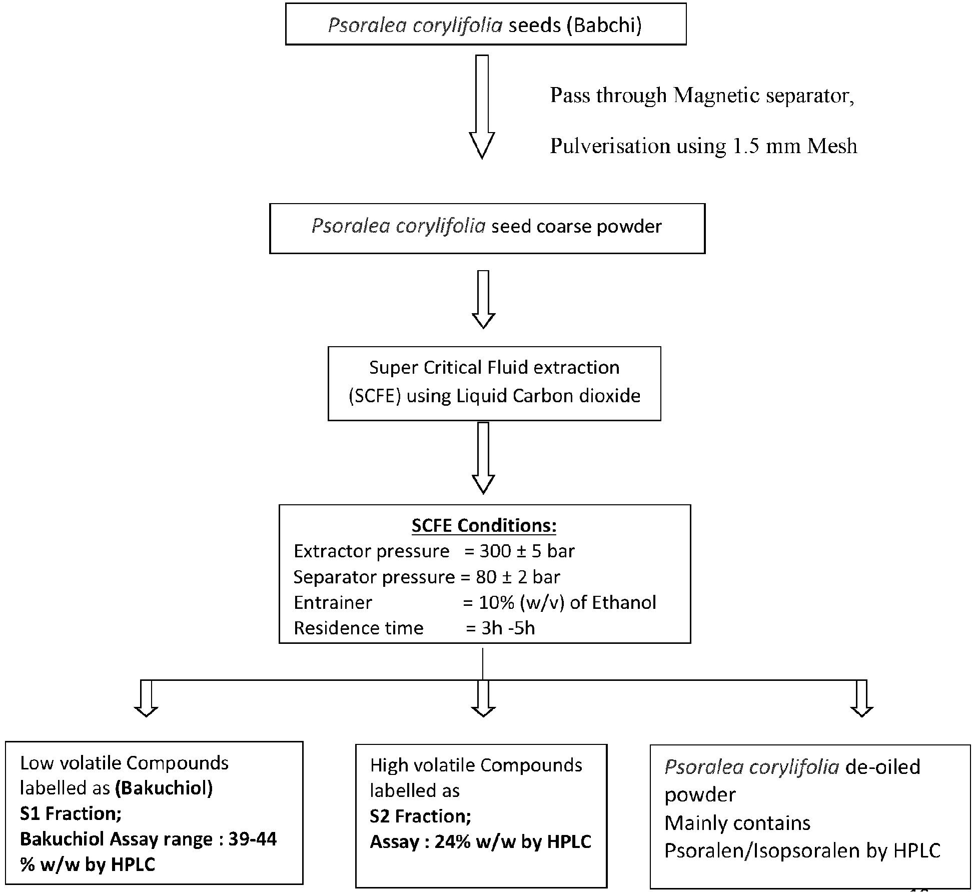

The content of Bakuchiol and Furanocoumarins is proportionately high in the S1 fraction. SCFE extraction is performed with the above condition and the cumulative results in each hour is described there in the below table -1.

**Table 1: SCFE extraction**

| | S1 FRACTION | | | S2 FRACTION |
|---|---|---|---|---|
| SCFE extraction | Extraction time 0-1 hour | Extraction time 0-2 hour | Extraction time 0-5 hour | |
| Input (100 Kg) | 55.1 Kg | 55.3 Kg | 51 Kg | 55 Kg |
| Description of the output | Yellowish brown coloured viscous oil with characteristic odour | Yellowish brown coloured viscous oil with characteristic odour | Yellowish brown coloured viscous oil with characteristic odour | Dark brownish oil |
| Assay Bakuchiol by HPLC % w/w | 44.23% | 43.28% | 39.88% | 24.63% |
| Psoralen | 2.63% | 3.06% | 3.9% | 42.69% |

From the above table it is evident that S1 fraction has the highest assay of Bakuchiol (between 39.88% to 44.23%) whereas in S2 fraction Bakuchiol content becomes proportionately reduced to 24.63%. However, the Psoralen content in S2 fraction is higher (42.69%).

After SCFE extraction, the fractions were subjected to High vacuum distillation (HVD) at desired temperature with controlled vacuum (66.7 Pa (0.5 mm Hg)) to obtain a bakuchiol extract free of psoralen and isopsoralen. The mechanism behind the distillation is probably to open the lactone ring of the furanocoumarin. Table 2 discloses the results of HVD.

**Table 2: High vacuum distillation of Psoralea corylifolia**

| | **Distillation Input Quantity : 55.1 Kg (Assay: 44.23% by HPLC)** | | | | |
|---|---|---|---|---|---|
| **Fraction Number** | **Still Temp (°C)** | **Vapour Temp (°C)** | **Vacuum (mm Hg)** | **Output (kg)** | **Bakuchiol Assay (% w/w)** |
| Fraction 1 | 195-233 | 150-192 | 0.5 | 6.06 | 66.02 |
| Fraction 2 | 233-250 | 192-197 | 0.5 | 4.35 | 61.70 |
| Fraction 3 | 250-265 | 198-205 | 0.5 | 1.65 | 78.13 |
| Bottom mass (pot residue) | | | | 40.22 | 2.70 |

The results indicate that Fractions 1,2 and 3 are enriched with bakuchiol and thus all the three fractions can be pooled together. The pot residue after pooling the fractions may generally be enriched with fatty acids with less bakuchiol and thus is discarded

Finally, the fractions from the HVD (from lower assay of bakuchiol 12.06 Kg, 66.15% average assay), are passed through Silica gel column chromatography which is prepacked with heptane and fractions are collected from 100% heptane to 2.0% Ethyl acetate/ heptane. Similar fractions were pooled to get higher content of pure bakuchiol (6.69 Kg, 99% w/w) (Table 3). Analysis is performed by high performance liquid chromatography (HPLC); Fraction details as given below in Table-3 :

**Table 3: Silica gel column purification**

| **Fractions Number** | **Eluted Solvent** | **Fractions quantity (kg)** | **Content of Bakuchiol % (w/w) by HPLC** |
|---|---|---|---|
| 1 to 11 | Heptane only | 0.12 | 70.18% |
| 12 to 16 | 0.5% (v/v) Ethyl acetate / heptane | 0.72 | 83.77% |
| 17 | 1% (v/v) Ethyl acetate / heptane | 0.89 | 99.05% |
| 18 | 1% (v/v) Ethyl acetate / heptane | 0.95 | |
| 19 | 1% (v/v) Ethyl acetate / heptane | 0.78 | 99.12% |
| 20 | 1% (v/v) Ethyl acetate / heptane | 0.61 | |
| 21 | 1% (v/v) Ethyl acetate / heptane | 1.34 | 99.26% |
| 22 | 1% (v/v) Ethyl acetate / heptane | 1.31 | 99.50% |
| 23 | 1% (v/v) Ethyl acetate / heptane | 0.25 | 99.63% |
| 24 | 2% (v/v) Ethyl acetate / heptane | 0.56 | 99.75% |
| 25 | 2% (v/v) Ethyl acetate / heptane | 0.56 | 58.12% |
| | Total quantity | 6.69 kg | 99% |

From the above fractions, fraction No. 17-24 (6.69 Kg of pure 99% bakuchiol) were collected separately by further purification and monitored using Thin layer chromatography [Mobile phase = Heptane: Ethyl acetate (7:3)] detection under UV wavelength 254 nm to yield a bakuchiol extract that is substantially free of furanocoumarins.

### Example 2: Formulations (comparative)

Tables 4-7 provide illustrative examples of nutraceutical and cosmeceutical formulations

**Table 4: Tablet**

| **Active Ingredients** |
|---|
| Bakuchiol (Babchiol^{™}) |

| **Excipients** |
|---|
| Microcrystalline cellulose, Colloidal silicon dioxide, Magnesium stearate, BioPerine^{®}, Polyvinylpyrrolidone / starch / Hydroxy propyl methyl cellulose, Hydroxy propyl cellulose |

**Table 5: Capsule**

| **Active Ingredients** |
|---|
| Bakuchiol (Babchiol^{™}) |

| **Excipients** |
|---|
| Microcrystalline cellulose, Colloidal silicon dioxide, Magnesium stearate, BioPerine^{®}, Polyvinylpyrrolidone / starch / Hydroxy propyl methyl cellulose, Hydroxy propyl cellulose |

**Table 6: Powder**

| **Active Ingredients** |
|---|
| Bakuchiol (Babchiol^{™}) |

| **Excipients** |
|---|
| BioPerine^{®}, Starch |

**Table 7 : Cream**

| **Phase** | **Ingredients** | **Functions** |
|---|---|---|
| A | Purified Water | Diluent |
| | Disodium EDTA | Chelating Agent |
| | Biopol Crystals | Thickener |
| | Glycerin | Humectant |
| B | Olivem 1000 | Emulsifier |
| | DUB CO | Emollient |
| | GMS SE | Emulsifier |
| | Cetyl Alcohol | Emollient |
| | Shea Butter | Emollient |
| | Imex IN3 | Emollient |
| | Tinogard TT | Anti Oxidant |
| | Tinogard TS | Anti Oxidant |
| C | Tween 20 | Solubilizer |
| | Tetrahydrocurcuminoids | Skin Lightening/Whitening |
| D | Purified Water | Diluent |
| | Bakuchiol (Babchiol^{™}) | Skin Lightening/Whitening |
| E | Euxyl PE 9010 | Preservative |
| | Xiameter PMX 3031 | Detackification |
| F | Purified Water | Diluent |
| | Sodium Metabisulfite | Anti Oxidant |

## Claims

1. A process of isolating pure bakuchiol (not less than 99% w/w) as represented in STR#1, substantially free of furanocoumarins from *Psoralea sp.,* comprising steps of:
a) Powdering *Psoralea* sp. plant parts and pulverizing using 1.5 mm mesh and passing through magnetic separator to obtain a coarse powder;
b) Subjecting the enriched extract of step a) to super critical fluid extraction (SCFE) using liquid CO₂ in the presence of an entrainer and a stabilizing agent to obtain two fractions: low volatile compounds-S1 fraction, high volatile compounds-S2 fraction and a spent material (residue);
c) Identifying the compounds in low volatile and high volatile fractions of step b) as bakuchiol, wherein the total yield of bakuchiol in S1 fraction is 5-15% w/w and 0.5 - 3% w/w in S2 fraction;
d) Pooling the S1 and S2 fractions from step b) to obtain bakuchiol enriched extract, substantially free of furanocoumarins;
e) Subjecting the bakuchiol enriched extract of step d) to high vacuum distillation (HVD) at desired temperature with vacuum to obtain different bakuchiol enriched fractions and a pot residue, wherein the bakuchiol content is assayed using HPLC;
f) Pooling the bakuchiol enriched fractions of step e) and passing them through a Silica gel column prepacked with heptane and eluting using a suitable mobile phase and collecting different fractions to get obtain content of bakuchiol (99% w/w) as assayed by HPLC; and
g) Collecting the fractions with bakuchiol content of not less than 99% w/w and further subjecting the fractions to Thin Layer chromatography with suitable solvents as mobile phase with detection under wavelength UV 254 nm, to obtain bakuchiol free or substantially free of furanocoumarins.

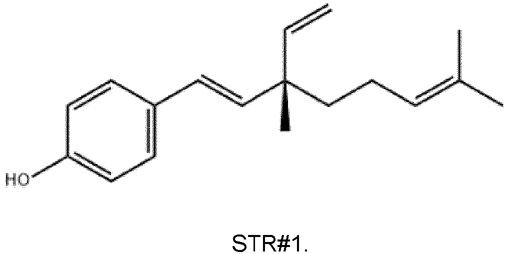

2. The process as in claim 1, wherein the furanocoumarins are psoralen and isopsoralen.

3. The process as in claim 1, wherein the stabilizing agent in SCFE is selected from the group consisting of rosmarinic acid, butylated hydroxyanisole, butylated hydroxytoluene, sodium metabisulfite, propyl gallate, cysteine, ascorbic acid and tocopherols.

4. The process as in claim 1, wherein the entrainer of step b) is selected from the group consisting of ethanol, methanol, isopropyl alcohol, acetone, chloroform, benzene, hexane, heptane, cyclohexane and combinations thereof.

5. The process as in claim 1, wherein the entrainer of step b) is selected from the group consisting of ethanol, methanol and isopropyl alcohol.

6. The process as in claim 1, wherein the mobile phase in step f) is selected from the group consisting of heptane and ethyl acetate or combinations thereof.

7. The process as in claim 1, wherein the mobile phase in step g) is selected from the group of n-heptane and ethyl acetate or combinations thereof, wherein the ratio of heptane and ethyl acetate is between 2-9 : 1-8.

## Patentansprüche

1. Verfahren zum Isolieren von reinem Bakuchiol (nicht weniger als 99 Gew.-%), wie in STR#1 dargestellt, im Wesentlichen frei von Furanocumarinen, aus *Psoralea* sp., umfassend die Schritte:
a) Pulvern von Pflanzenteilen von *Psoralea* sp. und Pulverisieren unter Verwendung eines 1,5-mm-Siebs und Durchleiten durch einen Magnetabscheider, um ein grobes Pulver zu erhalten;
b) Unterziehen des angereicherten Extrakts aus Schritt a) einer überkritischen Fluidextraktion (SCFE) unter Verwendung von flüssigem CO₂ in Gegenwart eines Schleppmittels und eines Stabilisierungsmittels, um zwei Fraktionen zu erhalten: schwerflüchtige Verbindungen - S1-Fraktion, leichtflüchtige Verbindungen - S2-Fraktion und ein verbrauchtes Material (Rückstand);
c) Identifizieren der Verbindungen in der schwerflüchtigen und der leichtflüchtigen Fraktion aus Schritt b) als Bakuchiol, wobei die Gesamtausbeute an Bakuchiol in der S1-Fraktion 5 - 15 Gew.-% und in der S2-Fraktion 0,5 - 3 Gew.-% beträgt;
d) Vereinigen der S1- und S2-Fraktionen aus Schritt b), um einen mit Bakuchiol angereicherten Extrakt zu erhalten, der im Wesentlichen frei von Furanocumarinen ist;
e) Unterziehen des mit Bakuchiol angereicherten Extrakts aus Schritt d) einer Hochvakuumdestillation (HVD) bei gewünschter Temperatur unter Vakuum, um verschiedene mit Bakuchiol angereicherte Fraktionen und einen Sumpfrückstand zu erhalten, wobei der Bakuchiolgehalt mittels HPLC bestimmt wird;
f) Vereinigen der mit Bakuchiol angereicherten Fraktionen aus Schritt e) und Durchleiten dieser durch eine mit Heptan vorgepackte Kieselgelsäule und Eluieren unter Verwendung einer geeigneten mobilen Phase und Sammeln verschiedener Fraktionen, um einen Gehalt an Bakuchiol (99 Gew.-%) zu erhalten, wie mittels HPLC bestimmt wird; und
g) Sammeln der Fraktionen mit einem Bakuchiolgehalt von nicht weniger als 99 Gew.-% und ferner Unterziehen der Fraktionen einer Dünnschichtchromatographie mit geeigneten Lösungsmitteln als mobiler Phase mit Nachweis bei einer Wellenlänge von UV 254 nm, um Bakuchiol frei oder im Wesentlichen frei von Furanocumarinen zu erhalten.

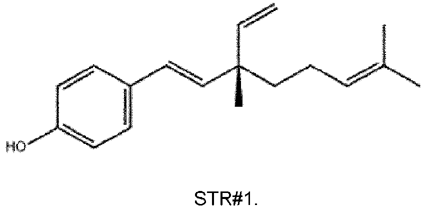

2. Verfahren wie in Anspruch 1, wobei die Furanocumarine Psoralen und Isopsoralen sind.

3. Verfahren wie in Anspruch 1, wobei das Stabilisierungsmittel in der SCFE ausgewählt ist aus der Gruppe bestehend aus Rosmarinsäure, butyliertem Hydroxyanisol, butyliertem Hydroxytoluol, Natriummetabisulfit, Propylgallat, Cystein, Ascorbinsäure und Tocopherolen.

4. Verfahren wie in Anspruch 1, wobei das Schleppmittel von Schritt b) ausgewählt ist aus der Gruppe bestehend aus Ethanol, Methanol, Isopropylalkohol, Aceton, Chloroform, Benzol, Hexan, Heptan, Cyclohexan und Kombinationen davon.

5. Verfahren wie in Anspruch 1, wobei das Schleppmittel von Schritt b) ausgewählt ist aus der Gruppe bestehend aus Ethanol, Methanol und Isopropylalkohol.

6. Verfahren wie in Anspruch 1, wobei die mobile Phase in Schritt f) ausgewählt ist aus der Gruppe bestehend aus Heptan und Ethylacetat oder Kombinationen davon.

7. Verfahren wie in Anspruch 1, wobei die mobile Phase in Schritt g) ausgewählt ist aus der Gruppe von n-Heptan und Ethylacetat oder Kombinationen davon, wobei das Verhältnis von Heptan und Ethylacetat zwischen 2 - 9 : 1 - 8 liegt.

## Revendications

1. Processus d'isolement du bakuchiol pur (à au moins 99 % m/m) tel que représenté par la formule STR#1, pratiquement exempt de furocoumarines, à partir de *Psoralea* sp., comprenant les étapes suivantes :
a) réduction en poudre de parties de la plante *Psoralea sp.* et pulvérisation à l'aide d'un tamis de 1,5 mm et en les passant à travers un séparateur magnétique pour obtenir une poudre grossière ;
b) soumission de l'extrait enrichi de l'étape a) à une extraction par fluide supercritique (EFS) en utilisant du CO₂ liquide en présence d'un agent d'entraînement et d'un agent de stabilisation pour obtenir deux fractions : des composés à faible teneur en composés volatils - fraction S1, des composés à forte teneur en composés volatils - fraction S2, et un matériau usé (résidu) ;
c) identification des composés dans les fractions à faible et à forte volatilité de l'étape b) comme étant le bakuchiol, dans lequel le rendement total de bakuchiol est de 5 à 15 % m/m dans la fraction S1 et de 0,5 à 3 % m/m dans la fraction S2 ;
d) regroupement des fractions S1 et S2 de l'étape b) pour obtenir un extrait enrichi en bakuchiol, pratiquement exempt de furocoumarines ;
e) soumission de l'extrait enrichi en bakuchiol de l'étape d) à une distillation sous vide poussé (DVP) à la température souhaitée sous vide pour obtenir différentes fractions enrichies en bakuchiol et un résidu de pot, dans lequel la teneur en bakuchiol est dosée par HPLC ;
f) regroupement des fractions enrichies en bakuchiol de l'étape e), puis passage sur une colonne de gel de silice pré-chargée en heptane, élution à l'aide d'une phase mobile appropriée et collecte des différentes fractions pour obtenir la teneur en bakuchiol (99 % m/m) dosée par HPLC ; et
g) collecte des fractions dont la teneur en bakuchiol est d'au moins 99 % m/m et soumission ensuite des fractions à une chromatographie sur couche mince avec des solvants appropriés comme phase mobile, avec détection sous une longueur d'onde UV de 254 nm, pour obtenir du bakuchiol exempt ou pratiquement exempt de furocoumarines.

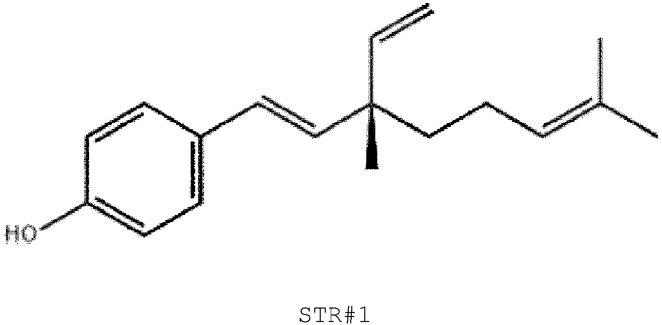

2. Processus selon la revendication 1, dans lequel les furocoumarines sont le psoralène et l'isopsoralène.

3. Processus selon la revendication 1, dans lequel l'agent de stabilisation pour la SCFE est sélectionné parmi le groupe constitué par l'acide rosmarinique, l'hydroxyanisole butylé, l'hydroxytoluène butylé, le métabisulfite de sodium, le gallate de propyle, la cystéine, l'acide ascorbique et les tocophérols.

4. Processus selon la revendication 1, dans lequel l'agent d'entraînement de l'étape b) est sélectionné parmi le groupe constitué par l'éthanol, le méthanol, l'alcool isopropylique, l'acétone, le chloroforme, le benzène, l'hexane, l'heptane, le cyclohexane, ainsi que les combinaisons de ceux-ci.

5. Processus selon la revendication 1, dans lequel l'agent d'entraînement de l'étape b) est sélectionné parmi le groupe constitué par l'éthanol, le méthanol et l'alcool isopropylique.

6. Processus selon la revendication 1, dans lequel la phase mobile de l'étape f) est sélectionnée parmi le groupe constitué par l'heptane, l'acétate d'éthyle, ainsi que les combinaisons de ceux-ci.

7. Processus selon la revendication 1, dans lequel la phase mobile de l'étape g) est sélectionnée parmi le groupe constitué par le n-heptane, l'acétate d'éthyle, ainsi que les combinaisons de ceux-ci, dans lequel le ratio entre l'heptane et l'acétate d'éthyle est compris entre 2,9 et 1,8.
